(19) **Europäisches Patentamt**
**European Patent Office**
**Office européen des brevets**

(11) Numéro de publication : **0 288 402 B1**

(12) **FASCICULE DE BREVET EUROPEEN**

(45) Date de publication du fascicule du brevet :
**23.10.91 Bulletin 91/43**

(51) Int. Cl.⁵ : **A61F 2/38**

(21) Numéro de dépôt : **88420132.8**

(22) Date de dépôt : **22.04.88**

(54) **Prothèse bi-compartimentaire d'articulation du genou.**

(30) Priorité : **23.04.87 FR 8706043**

(43) Date de publication de la demande :
**26.10.88 Bulletin 88/43**

(45) Mention de la délivrance du brevet :
**23.10.91 Bulletin 91/43**

(84) Etats contractants désignés :
**AT BE CH DE ES GB GR IT LI LU NL SE**

(56) Documents cités :
**DE-A- 2 660 623**
**US-A- 4 211 228**

(73) Titulaire : **Broc, Christian**
**Les Beaumettes**
**F-84220 Gordes (FR)**

(72) Inventeur : **Broc, Christian**
**Les Beaumettes**
**F-84220 Gordes (FR)**

(74) Mandataire : **Dupuis, François**
**Cabinet Laurent et Charras 3, place de**
**l'Hôtel-de-Ville**
**F-42000 Saint-Etienne (FR)**

Jouve, 18, rue Saint-Denis, 75001 PARIS

## Description

L'invention concerne une prothèse bi-compartimentaire d'articulation du genou.

L'objet de l'invention se rattache au secteur technique des sciences médicales.

Pour remettre un genou opéré dans ces conditions biomécaniques optimales, différents types de prothèses peuvent être employés. Généralement, on distingue les prothèses, tri-compartimentaires, bi-compartimentaires et mono-compartimentaires.

L'invention vise plus particulièrement, quoique non rigoureusement limitativement, les prothèses du type bi-compartimentaires comprenant pour l'essentiel un élément fémoral conformé pour être adapté à l'anatomie du ou des condyles fémoraux et destiné à coopérer en appui sur un élément tibial que l'on fixe sur les coupes tibiales effectuées.

L'élément tibial se compose de deux plateaux en polyéthylène ou autre, de différents diamètres et épaisseurs, en étant conformés pour être adaptés au mieux au profil du compartiment interne et/ou externe résultant des coupes tibiales à 90°. A noter que les plateaux peuvent être posés directement sur les compartiments ou par l'intermédiaire d'embases support. Les plateaux de l'élément tibial peuvent être accouplés par un élément commun ou bien être totalement indépendants.

Dans le cas de plateaux tibiaux non-indépendants, l'élément de jonction oblige souvent de sacrifier une partie des composantes de l'articulation du genou. A l'inverse, dans le cas de plateaux indépendants, un problème important apparaît au niveau de leur mise en place. En effet, il est nécessaire que la surface d'appui de chacun des plateaux soit dans le même plan horizontal pour répartir régulièrement les efforts.

D'autre part, à ce jour, les éléments prothétiques proposés ne tiennent pas compte des composantes rotatoires résultant de la flexion du genou au niveau du compartiment externe qui est celui de la mobilité. En effet, dans un genou normal, les ligaments latéraux se détendent en flexion et la plupart des mouvements de rotation se font sur le côté, le centre de rotation étant le condyle interne.

De telles prothèses sont donc semi-contraintes en étant susceptibles d'entraîner des liaisons plus ou moins importantes, avec des risques de descellement, les condyles demeurent contraints.

Pour remédier à ces inconvénients et répondre aux conditions anatomiques de l'articulation du genou, l'invention s'est fixée pour but, d'une manière simple, efficace et rationnelle de réaliser un nouveau type de prothèse dont l'élément tibial est composé de deux plateaux indépendants, et conformé et agencé avec des moyens pour, d'une part, assurer le parfait alignement horizontal des deux surfaces d'appui de chacun des plateaux et, d'autre part, tenir compte des composantes de rotation lors du mouvement de flexion-extension du genou.

A cet effet, selon une première caractéristique, le plateau tibial interne présente directement ou d'une manière rapportée, un moyen de guidage et de positionnement coopérant avec une entaille complémentaire formée antérieurement dans l'épaisseur du massif des épines tibiales en étant susceptible de déborder au-delà dudit massif dans le compartiment externe, pour coopérer avec un moyen de guidage et de positionnement complémentaire recevant, à libre rotation, le plateau tibial externe, pour positionner ultérieurement sa surface d'appui dans le même plan horizontal que celui du plateau interne, ledit moyen de guidage et de positionnement du plateau interne étant conformé pour recevoir un organe de repérage susceptible de coopérer avec le bord interne du condyle externe à 90° de flexion du genou pour indiquer la coupe angulaire à effectuer sur le côté correspondant du massif des épines tibiales en fonction de la rotation dudit genou, en vue d'assurer le positionnement angulaire du plateau externe en appui sur le côté réséqué du massif.

Ainsi conformé, l'élément tibial de prothèse tient compte d'une manière avantageuse, de l'asymétrie de taille du plateau externe par rapport au plateau interne, de l'asymétrie rotatoire et de l'asymétrie de profondeur des compartiments interne et externe.

L'invention est exposée ci-après plus en détail à l'aide des dessins qui représente seulement un mode d'exécution :

— la figure 1 est une vue en perspective des principaux éléments de l'implant tibial,

— les figures 2 à 9 sont des vues à caractère purement schématique, de face et en plan, montrant les principales phases opératoires de mise en place de l'élément tibial de prothèse,

— la figure 10 est une vue de face montrant une prothèse bi-compartimentaire avec l'élément tibial selon l'invention.

Afin de rendre plus concret l'objet de l'invention, on le décrit maintenant d'une manière non limitative en se référant aux exemples de réalisation des figures des dessins.

D'une manière connue, l'élément tibial se compose de deux plateaux indépendants (1) et (2) correspondant respectivement aux compartiments interne (I) et externe (E). Chaque plateau, généralement réalisé en polyéthylène, est exécuté en plusieurs diamètres et épaisseurs. La face inférieure peut être quadrillée ou coopérer avec une embase d'appui métallique et rapportée. La face supérieure coopérant avec la partie du condyle de l'élément fémoral est très légèrement concave aussi bien dans le plan latéral que transversal.

Selon l'invention, le plateau interne (1) reçoit directement ou d'une manière rapportée, un moyen de guidage et de positionnement (3) sous forme d'une partie de lunette. Cette partie de lunette (3) présente une branche (3a) en débordement de la face rectiligne du plateau (1) et destinée à coopérer avec une entaille complémentaire (A) formée facialement dans l'épaisseur du massif des épines tibiales, du côté antérieur. La longueur de la branche (3a) est telle qu'elle déborde au-delà du massif des épines tibiales, dans le compartiment externe.

Cette partie de lunettes (3) peut être fixée temporairement ou à demeure avec le bord périphérique du plateau tibial interne (1) par tout moyen connu et approprié. L'extrémité de la lunette (3) opposée à la branche (3a), est raccordée avec la périphérie du plateau (1) au niveau de l'intersection avec la médiane dudit plateau.

L'extrémité débordante de la branche (3a) de lunette (3), coopére avec une branche (4a) d'une lunette complémentaire (4) susceptible d'être adaptée autour du plateau tibial externe (2) en laissant libre en rotation ledit plateau. L'accouplement des branches des lunettes (3) et (4) peut s'effectuer d'une manière réglable, pour faire varier la longueur d'écartement entre les différentes lunettes. Cet accouplement peut s'effectuer par tout moyen connu et approprié, par exemple, par coulissement d'un agencement de l'extrémité de la branche (3a) dans un agencement complémentaire formé en bout de la branche (4a) de l'autre partie de la lunette complémentaire (4).

De la même façon que pour la lunette (3), l'extrémité de la lunette (4) opposée à la branche (4a), se raccorde avec le bord périphérique du plateau (2) au niveau de l'intersection de sa ligne médiane.

D'une manière importante, après positionnement des parties de lunettes (3) et (4) dans les agencements correspondants des plateaux tibiaux interne et externe, la distance entre la partie supérieure de chacune des lunettes (3) et (4) et la face d'appui des plateaux recevant les condyles est très rigoureusement la même. A noter du reste, que cette distance peut être nulle. Il en résulte donc, qu'après avoir positionné le plateau tibial interne (1) avec sa lunette (3), on est certain que la surface d'appui du plateau tibial externe (2), compte-tenu de l'accouplement des deux parties de lunettes (3) et (4), sera très exactement dans le même plan horizontal que celui de la surface d'appui du plateau interne.

La branche antérieure (3a) de la partie de lunette (3), peut présenter, en bout, très sensiblement au niveau de l'axe médian du massif des épines tibiales, un plot (3c) susceptible de recevoir verticalement une broche (5) pour vérifier indirectement l'horizontalité de ladite lunette (3) et par conséquent du plateau (1).

Suivant une autre caractéristique, la branche antérieure (3a) de la lunette présente très sensiblement, au niveau de son extrémité libre, une lumière horizontale (3d) ou autre agencement permettant l'engagement d'une broche (6) susceptible de coopérer en appui avec le bord interne du condyle externe pour faire office de repère en vue de déterminer l'angle de coupe du massif des épines tibiales, du côté du compartiment externe.

La pose de ce type particulier d'implant tibial s'effectue comme suit :

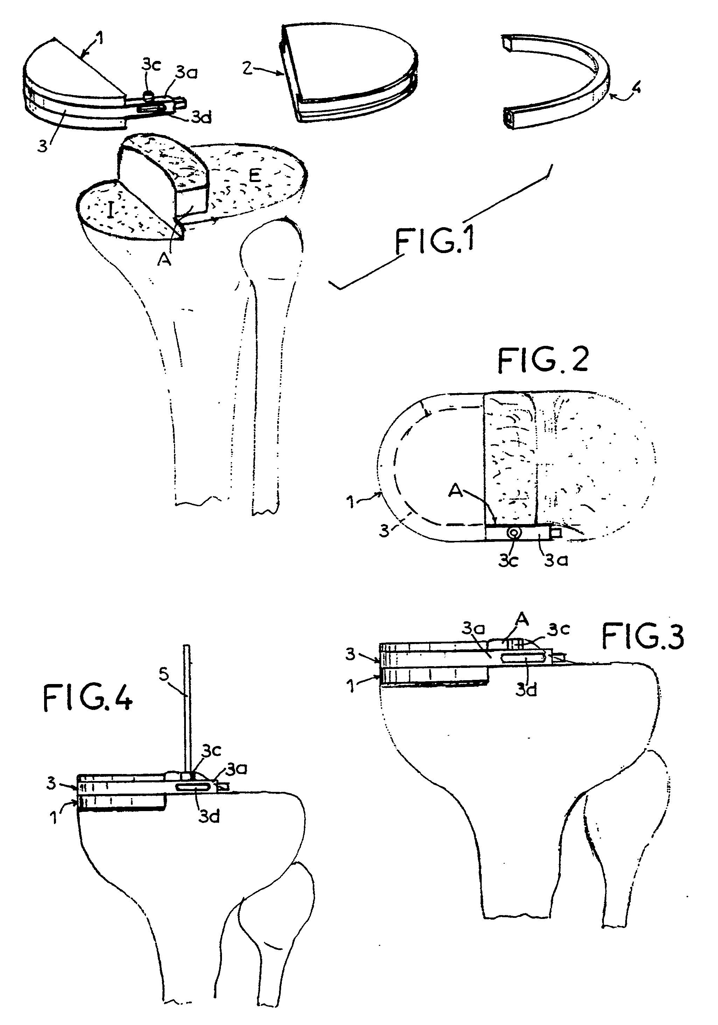

On effectue, dans un premier temps, d'une manière classique et par tout moyen connu et approprié à la portée du spécialiste, la ou les coupes tibiales à 90° de flexion, puis on exécute l'entaille (A) dans le massif des épines pour l'engagement de la branche (3a) de la lunette (3). On positionne alors le plateau tibial interne (1) dans le compartiment correspondant. La branche (3a) de la lunette (3), engagée et positionnée dans l'entaille (A) du massif, déborde très légèrement au niveau du compartiment externe (figure 2 et 3).

On vérifie au moyen de la broche (5) que l'on engage verticalement dans le plot (3c), le niveau horizontal du plateau interne (1) (figure 4).

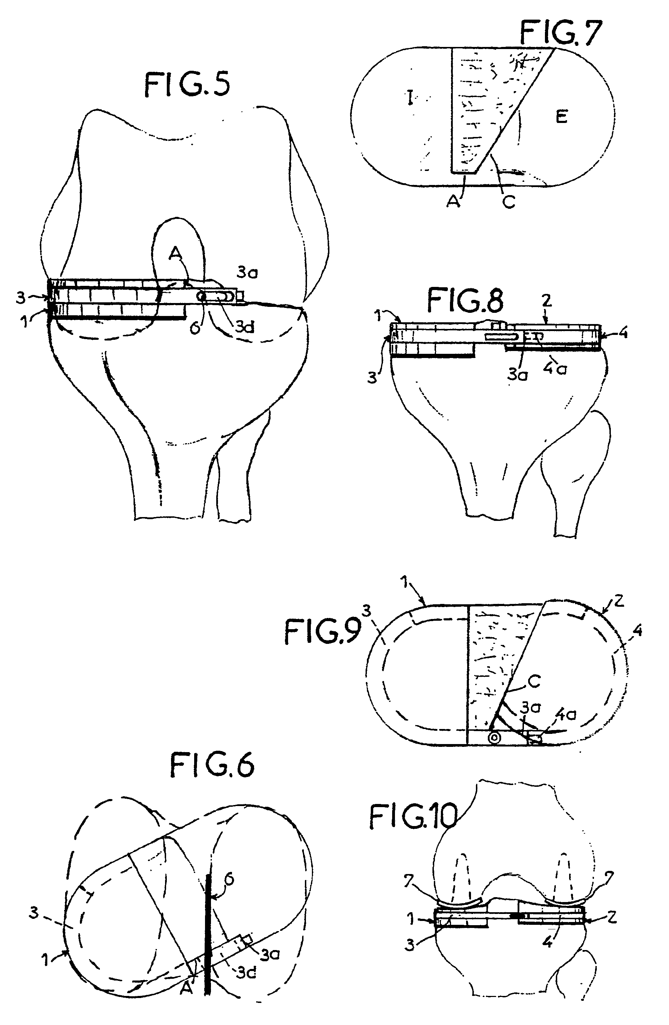

On met alors le genou à 90° de flexion ce qui engendre un effet rotationnel du tibia par rapport aux condyles fémoraux (figure 5 et 6) étant rappelé que le condyle interne est le centre de la rotation.

On engage ensuite le guide broche (6) dans la lumière (3d) de la branche (3a) de manière à le faire tangenter au bord axial interne du condyle externe faisant ainsi office de repère pour le plan de coupe angulaire (C) du massif (figure 6). La broche (6) demeurant positionnée angulairement dans la lunette, on remet le genou en extension de sorte que l'on obtient directement la coupe verticale qui s'effectue à environ 30° sur le massif des épines tibiales, du côté du compartiment externe (figure 7).

Après avoir effectué la deuxième coupe tibiale du compartiment interne, dans le même plan horizontal que celui défini par la lunette (3), il suffit de mettre en place le plateau tibial externe (2) en appui sur le bord réséqué du massif, puis d'adapter autour dudit plateau la partie correspondante de lunette (4) et d'accoupler les branches (3a) et (4a) (figure 8). La lunette (4) coopérant avec la lunette (3) du compartiment interne (1) assure donc au plateau externe (2), d'une part, le positionnement de sa surface d'appui dans le même plan horizontal que celui du compartiment interne et, d'autre part, le maintien en position angulaire dudit compartiment (2) par rapport au massif des épines tibiales (figure 9).

Les figures 8 et 9 notamment, montrent clairement que l'élément prothétique tibial et son ancillaire de pose tiennent compte de l'asymétrie de taille du plateau externe (2) par rapport au plateau interne (1), de l'asymétrie rotatoire résultant de la flexion du genou et enfin de l'asymétrie de profondeur des compartiments interne (I) et externe (E).

L'élément prothétique fémoral (7) coopérant avec les plateaux tibiaux (1) et (2) n'est pas décrit car il ne fait pas partie de l'objet spécifique du brevet. Cet élément fémoral peut être monobloc ou bi-bloc. On renvoie à la figure 10 qui illustre un exemple de prothèse adaptée au genou.

Les avantages ressortent bien de la description. En particulier, on souligne que l'on obtient une prothèse non contrainte étant donné que les composantes rotatoires du genou sont prises en compte par le positionnement angulaire du plateau externe. Ces dispositions originales sont en outre combinées avec le fait d'utiliser deux plateaux tibiaux indépendants avec la certitude de placer les surfaces d'appui desdits plateaux selon un même plan horizontal pour assurer la répartition des efforts.

Les lunettes (3) et (4) sont exécutées en tous matériaux appropriés, notamment en titane.

La hauteur du plateau peut être variable en respectant toutefois de garder constante la distance entre les lunettes et les surfaces d'appui pour le condyle. De même, différents moyens peuvent être employés pour assurer l'accouplement et le réglage dimensionnel des branches de lunettes (3) et (4). On prévoit d'équiper une partie de la lunette (3) notamment d'un plot de fixation coopérant avec une partie correspondante du tibia, pour assurer une meilleure rigidité de l'ensemble.

## Revendications

1. Prothèse bi-compartimentaire du genou comprenant un élément fémoral (7) et un élément tibial sous forme de deux plateaux indépendants (1, 2), caractérisée en ce que le plateau tibial interne (1) présente directement ou d'une manière rapportée, un moyen de guidage et de positionnement (3) dont une partie coopère avec une entaille complémentaire (A) formée antérieurement dans l'épaisseur du massif des épines tibiales en étant susceptible de déborder au-delà dudit massif dans le compartiment externe, ladite partie étant agencée pour recevoir un organe de repérage (6) susceptible de coopérer avec le bord interne du condyle externe à 90° de flexion du genou pour indiquer une coupe angulaire (C) à effectuer sur le côté correspondant du massif des épines tibiales en fonction de l'effet rotationnel produit du tibia par rapport aux condyles fémoraux, en vue d'assurer le positionnement angulaire du plateau externe (2) en appui sur le côté réséqué du massif, ledit plateau externe étant agencé pour recevoir avec capacité de réglage angulaire, un moyen de guidage et de positionnement complémentaire (4) susceptible de coopérer avec le moyen (3) pour positionner la surface d'appui de chacun des plateaux interne et externe dans le même plan horizontal.

2. Prothèse selon la revendication 1, caractérisée en ce que les moyens de positionnement et de guidage (3) et (4) sont constitués par des parties de lunettes.

3. Prothèse selon la revendication 2, caractérisée en ce que la partie de lunette (3) présente une branche (3a) en débordement de la face rectiligne du plateau (1) et destinée à être engagée dans l'entaille (A), l'extrémité opposée à ladite branche (3a) étant raccordée avec la périphérie du plateau (1) au niveau de l'intersection de sa ligne médiane.

4. Prothèse selon la revendication 3, caractérisée en ce que l'extrémité de la branche (3a) est conformée pour être reliée à une partie correspondante d'une branche (4a) de la lunette (4) en vue d'assurer l'accouplement réglable des lunettes (3) et (4).

5. Prothèse selon la revendication 2, caractérisée en ce qu'après positionnement des parties de lunettes (3) et (4) dans des agencements correspondant des plateaux tibiaux interne et externe (1) et (2), la distance entre chacune desdites lunettes (3) et (4) et la face d'appui desdits plateaux, est la même.

6. Prothèse selon les revendications 1 et 2 ensemble, caractérisée en ce qu'une partie de la branche (3a) de la lunette (3) présente, au niveau du massif des épines tibiales, une lumière horizontale (3d) pour l'engagement de l'organe de repérage (6).

7. Prothèse selon les revendications 1 et 2 ensemble, caractérisée en ce qu'une partie de la branche antérieure (3a) de la lunette (3) présente un plot pour l'engagement vertical d'une broche (5) en vue de contrôler l'horizontalité de cette lunette.

8. Prothèse selon les revendications 1 et 2 ensemble, caractérisée en ce que la lunette (4) est conformée en section pour recevoir le plateau externe (2) avec capacité de pivotement circulaire.

## Claims

1. Bi-compartment knee prosthesis comprising a femoral component (7) and a tibial component in the form of two independent plateaux (1, 2) wherein the internal tibial plateau (1) has either directly or in a built up manner, a guiding and positioning means (3), one part of which cooperates with a complementary notch (A) formed in the front of the tibial ridge bed being likely to project beyond the maid bed in the external compartment, the said part being designed to take a locating component (6) likely to cooperate with the internal edge of the external condyle at a 90° of flexion of the knee in order to indicate an angular cut (C) to be made on the corresponding end of the tibial ridge bed as a function of the rotational effect produced from the tibia with respect to the femoral condyles, with a view to ensuring the angular positioning of the external plateau (2) applied against the resected end of the bed, the said external plateau being designed to take, capable of angular adjustment, complementary guiding and positioning means (4) likely to cooperate with the means (3) to position the bearing surface of each of the internal and external plateaux in the same horizontal plane.

2. Prosthesis according to claim 1, wherein the positioning and guiding means (3) and (4) are made up of parts of segments.

3. Prosthesis according to claim 2, wherein the segment part (3) has a leg (3a) projecting from the rectilinear face of the plateau (1) and designed to be inserted into the notch (A), the opposite end to the said leg (3a) being connected with the periphery of the plateau (1) at the intersection of its medial line.

4. Prosthesis according to claim 3, wherein the end of the leg (3a) is designed to be connected to a corresponding part of a leg (4a) of the segment (4) with a view to ensuring the adjustable coupling of segments (3) and (4).

5. Prosthesis according to claim 2, wherein after positioning the segment parts (3) and (4) in the corresponding recesses of the internal and external tibial plateaux (1) and (2), the distance between each of the said segments (3) and (4) and the bearing surface of the said plateaux, is the same.

6. Prosthesis according to claims 1 and 2 together, wherein one part of the leg (3a) of the segment (3) has a horizontal slot (3d) at the tibial ridge bed for the locating component (6) to be inserted.

7. Prosthesis according to claims 1 and 2 together, wherein one part of the front leg (3a) of the segment (3) has a hole for the vertical insertion of a pin (5) with a view to checking the horizontality of this segment.

8. Prosthesis according to claims 1 and 2 together, wherein the section of the segment (4) is designed to take the external plateau (2), capable of circular pivoting.


## Patentansprüche

1. Die Zwei-Komponenten-Knieprothese umfaßt ein Femurteil (7) sowie ein Tibialteil in Form von zwei einzelnen Platten (1, 2), dadurch gekennzeichnet, daß die innere Tibialplatte (1) direkt oder in aufgesetzter Form ein Führungs- und Positionierteil (3) darstellt, dessen einer Teil in eine zusätzliche Einkerbung (A) greift, die vorher in die Dicke des Massivmaterial der Tibialdorne eingeformt wurde, wobei diese über das genannte massive Teil in die äußere Komponente hinausragen kann. Dabei wird das genannte Teil so angeordnet, daß es ein Markierungselement (6) aufnehmen kann, das mit dem inneren Rand des Höckers bei einer 90°-Biegung des Knies zusammenwirken kann, um einen Winkelschnitt (C) anzuzeigen, der auf der entsprechenden Seite des Massivmaterials der Tibialstifte entsprechend der erzeugten Drehwirkung des Tibials in bezug auf die Femur-höcker auszuführen ist, um die Winkelpositionierung der äußeren Platte (2), die auf der resezierten Seite des Massifteils aufliegt, sicherzustellen, wobei die genannte äußere Platte so angeordnet ist, daß sie mit der Möglichkeit der Winkelverstellung ein zusätzliches Führungselement (4) aufnehmen kann, das mit dem Teil (3) zusammenwirkt, um die Auflagefläche jeder der internen und externen Platten in gleicher Ebene positionieren zu können.

2. Prothese gemäß Anspruch 1, dadurch gekennzeichnet, daß die Positionier- und Führungselemente (3) und (4) durch Stützlagerteile gebildet werden.

3. Prothese gemäß Anspruch 2, dadurch gekennzeichnet, daß das Stützlagerteil (3) einen Schenkel (3a) umfaßt, die über die geradlinige Fläche der Platte (1) hinausragt und in die Einkerbung (a) eingreifen, wobei das gegenüberliegende Ende des genannten Schenkels (3a) in der Schnitthöhe seiner Mittellinie mit dem Außenrand der Platte (1) verbunden ist.

4. Prothese gemäß Anspruch 3, dadurch gekennzeichnet, daß das Ende des Schenkels (3a) so angepaßt ist, daß es mit einem entsprechenden Teil des Schenkels (4a) des Stützlagerteiles (4) verbunden werden kann, damit eine verstellbare Kupplung der Stützlagerteile (3) und (4) sichergestellt wird.

5. Prothese gemäß Anspruch 2, dadurch gekennzeichnet, daß nach der Positionierung der Stützlagerteile (3) und (4) in den entsprechenden Anordnungen der inneren und äußeren Tibialplatte (1) und (2) der Abstand zwischen jedem der genannten Stützlagerteile (3) und (4) und der Auflagefläche der genannten Platten gleich ist.

6. Prothese gemäß den Ansprüchen 1 und 2, dadurch gekennzeichnet, daß ein Teil des Schenkels (3a) des Stützlagerteiles (3) im Bereich des Massivteiles der Tibialstifte ein horizontales Stützlagerteil (3d) für den Eingriff des Markierungsorganes (6) aufweist.

7. Prothese gemäß den Ansprüchen 1 und 2, dadurch gekennzeichnet, daß ein Teil des vorderen Schenkels (3a) des Stützlagerteiles (3) einen Anschlag für den vertikalen Eingriff einer Spindel (5) zur Kontrolle der Horizontallage dieses Stützlagerteiles aufweist.

8. Prothese gemäß den Ansprüchen 1 und 2, dadurch gekennzeichnet, daß das Stützlagerteil (4) als Profil ausgebildet ist, um die äußere Platte (2) mit der Möglichkeit der kreisförmigen Drehung aufnehmen zu können.

FIG.1

FIG.2

FIG.4

FIG.3

FIG.7

FIG.5

FIG.8

FIG.9

FIG.6

FIG.10